# EUROPEAN PATENT APPLICATION

(11) **EP 1 541 153 A1**
(43) Date of publication of application: **15.06.2005**
(21) Application number: 03797666.9
(22) Date of filing: 18.09.2003
(51) Int. Cl.: A61K 31/7028, A61K 31/7032, A61P 1/16, A61P 31/14

(54) **HEPATITIS C VIRUS INHIBITOR COMPRISING ALPHA-GLYCOSYLCERAMIDE AS THE ACTIVE INGREDIENT**

(30) Priority: 20.09.2002 JP 2002275466
(71) Applicant: KIRIN BEER KABUSHIKI KAISHA, Tokyo 104-8288 (JP)
(72) Inventor: SERIZAWA, I. Pharmaceutical Research Laboratories, Takasaki-shi, Gunma 370-1295 (JP); USHIDA, K. Pharmaceutical Development Laboratories, Maebashi-shi, Gunma 371-0853 (JP); NISHI, Nobusuke Pharmaceutical Division, Shibuya-ku, Tokyo 150-8011 (JP)
(74) Representative: HOFFMANN - EITLE
(86) International application number: PCT/JP2003/011908
(87) International publication number: WO 2004/026318

(57) **Abstract**

This invention provides a growth inhibitor of human hepatitis C virus comprising, as an active ingredient, α-glycosylceramide used for patients infected with the aforementioned viruses. This inhibitor of hepatitis C virus comprises, as an active ingredient, a compound represented by formula (I) or a salt or solvate thereof.

## Description

### Technical Field

The present invention relates to a growth inhibitor of human hepatitis C virus comprising, as an active ingredient, α-glycosylceramide used for patients infected with the aforementioned viruses.

### Background Art

Recently, KRN7000, which had been synthesized as a derivative of natural sponge-derived agelasphins (Tetrahedron Lett., 34: 5591-5592, 1993; Tetrahedron Lett., 34: 5593-5596, 1993; Tetrahedron, 50: 2771-2784, 1994), was found to be a ligand for an invariant T cell receptor (TCR) expressed in natural killer T (NKT) cells (Science, 278: 1626-1629, 1997; J. Exp. Med., 188: 1521-1528, 1998; J. Exp. Med. 188: 1529-1534, 1998). α-Glycosylceramide (α-GlyCer) as represented by KRN7000 was reported to be a glycosphingolipid in which hydrophilic saccharides, such as galactose or glucose, bound to hydrophobic ceramides comprising fatty acids and sphingosine bases at the α position, that exhibited potent anti-tumor activity by stimulating NKT cells as a ligand (Oncol. Res., 7: 529-534, 1995; Cancer Res., 58: 1202-1207, 1998). α-GlyCer is first incorporated into an antigen-presenting cell (APC) including a dendritic cell (DC) as a glycolipid antigen, processed without involving the transporter associated with antigen processing (TAP) path, which is known to play a key role in protein antigen processing, and then presented by a major histocompatibility complex (MHC) antigen class Ib-like nonpolymorphic molecule, CD1d, on an APC. NKT cells become activated when KRN7000, which has been presented by CD1d on the APC, is recognized by an invariant TCR on the membrane of NKT cells. Based on this principle, human NKT cells can be efficiently cultured utilizing human monocyte-derived DCs to which α-GlyCer has been incorporated (Hum. Immunol., 60: 10-19, 1999). Further, interleukin-7 (IL-7) or IL-15 was reported to proliferate NKT cells *in vitro* synergistically with α-GlyCer (Hum. Immunol., 61: 357-365, 1999).

NKT cells are a type of lymphocytes and are unique in that they express both TCR and natural killer (NK) cell markers (Annu. Rev. Immunol., 15, 535-562, 1997; J. Exp. Med., 182, 633-638, 1995). One notable property is that the TCRα chain expressed in the NKT cells is invariant, and this α chain expresses an extremely limited TCR repertoire (invariant TCR) by pairing with a very limited type of β-chain. This stable TCRα chain is known as Vα24 in the case of humans and as Vα14 in the case of mice. The invariant TCR has been shown to be highly homologous between the two species. In particular, this TCRα exhibits very high homology of 85% in terms of amino acid in the CDR3 domain that is considered to be the central portion of the TCR antigen-binding site. The TCRβ chain is Vβ11 in the case of humans and Vβ8, Vβ7, or Vβ2 in the case of mice (J. Exp. Med., 180: 1097, 1994; Proc. Natl. Acad. Sci. USA, 88: 7518, 1991; Proc. Natl. Acad. Sci. USA, 89: 6506, 1992; J. Exp. Med., 176: 269, 1992). In contrast, NKR-P1 (CD161 or NK1.1) is expressed on the NKT cell as an NK cell marker, although its function has not yet been elucidated. The NKT cell can produce interferon-γ (IFN-γ), which is a Th1 type cytokine (a cytokine produced from the TH1 cell (Th1)), and interleukin-4 (IL-4), which is a Th2 type cytokine (the cytokine produced from the TH2 cells (Th2)) (J. Exp. Med., 179: 1285-1295, 1994; J. Exp. Med., 186: 109, 1997; J. Immunol., 161: 3271-3281, 1998). This indicates that the NKT cell is deeply involved with differentiation and regulation of T helper cells. Specifically, excessive production of IFN-γ could induce differentiation of the T helper cell into Th1 and excessive production of IL-4 could induced differentiation thereof into Th2. Further, NKT cells are reported to produce perforin and granzyme that play major roles in NK cell-like tumoricidal activity (Proc. Natl. Acad. Sci. USA, 95: 5690-5693, 1998; Hum. Immunol., 61: 357-365, 1999).

KRN7000 is known to act as a ligand of the NKT cell (Kawano, T. et al., Science 278: 1626, 1997). More specifically, KRN7000 is first incorporated into an antigen-presenting cell (APC) such as a dendritic cell (DC) and then presented onto the surface of the DC by an antigen-presenting molecule, CD1d. An invariant T cell receptor (TCR) expressed in the NKT cell can recognize a so-called immune complex consisting of CD1d and KRN7000 presented on the surface of the DC. The NKT cell bound to the immune complex is activated, and it induces a variety of immune responses.

β-Galactosylceramide, β-glycosylceramide, and the like in which a variety of saccharides are β-bound to ceramides are present *in vivo* (Svennerholm, L. et al., Biochem. Biophys. Acta, 280, 626, 1972; Karlsson, K.-A. et al., Biochim. Biophys. Acta, 316, 317, 1973). α-Galactosylceramide is known to have significant immunopotentiating action and antitumor action (Morita, M. et al., J. Med. Chem., 38, 2176, 1995). Also, the actions of α-galactosylceramide or α-glycosylceramide are known to be much more potent than those of β-galactosylceramide or β-glycosylceramide (Motoki, K. et al., Biol. Pharm. Bull., 18, 1487, 1995). Further, when compounds having α-glycosylceramide structures such as α-galactosylceramide or α-glycosylceramide are administrated in bodies, these compounds exhibit radioprotective action (Motoki, K. et al., Bioorg. Med. Chem. Lett., 5, 2413, 1995), inhibitory action against pulmonary metastasis of murine melanoma B16 (Kobayashi, E. et al., Oncology Res., 7, 529, 1995), and inhibitory action against liver metastasis of murine colon carcinoma cells (Colon 26) or murine T lymphoma EL4 (Motoki, K. et al., the Report of the Annual Meeting of the Japanese Cancer Association, 523, 1996). Such compounds are also known to increase the number of platelets or leucocytes (Motoki, K. et al., Biol. Pharm. Bull., 19, 952, 1996).

Applicability of α-galactosylceramide or α-glycosylceramide including KRN7000 as a therapeutic agent for infectious diseases is also suggested (WO 93/05055). Actually, when such a substance was administered to a human hepatitis B virus (HBV) transgenic mouse, HBV DNA replication in the liver of the mouse was potently inhibited (J. Exp. Med. 192: 921-930, 2000).

Even though the aforementioned substance had the effect of inhibiting HBV, this effect cannot be directly applied to other types of viruses. In particular, even through the effects of the substance thereon had been recognized, since HBV is a DNA virus, it cannot be said that the aforementioned substance can also affect RNA viruses such as human hepatitis C viruses (HCV).

At present, it is deduced that more than 100,000,000 people are infected with hepatitis C viruses (HCV) in the world. In many cases, HCV leads to chronic liver diseases, i.e., chronic hepatitis C. Further, there is a risk of developing hepatic cirrhosis, hepatocellular carcinoma, and the like. Hepatitis C viruses are roughly classified into 6 genotypes. In Japan, the United States, and Europe, approximately 70% of patients of hepatitis C are infected with genotype 1 (which is further classified into genotypes 1a and 1b). Interferon (IFN) has been a clinically approved therapeutic agent for patients of chronic hepatitis C. However, genotype 1 is known to apparently become resistant against the interferon therapy. Disadvantageously, interferon has a low rate of chronic responses and requires frequent dosage. Interferon therapy would induce side effects that would shorten the lives of patients who had been subjected to interferon therapy (i.e., retinopathy, thyroiditis, acute pancreatitis, or depression). Suicide resulting from depression is particularly a serious issue of concern. In the past, there was no agent that could be effectively used for inhibiting hepatitis C viruses. Accordingly, development of a novel agent for inhibiting hepatitis C viruses that can be effectively used for treating HCV infectious diseases has been desired.

Patent Document 1
WO 93/05055
Non-Patent Document 1
Tetrahedron Lett., 34: 5591-5592, 1993
Non-Patent Document 2
Tetrahedron Lett., 34: 5593-5596, 1993
Non-Patent Document 3
Tetrahedron, 50: 2771-2784, 1994
Non-Patent Document 4
Science, 278: 1626-1629, 1997
Non-Patent Document 5
J. Exp. Med., 188: 1521-1528, 1998
Non-Patent Document 6
J. Exp. Med. 188: 1529-1534, 1998
Non-Patent Document 7
Oncol. Res., 7: 529-534, 1995
Non-Patent Document 8
Cancer Res., 58: 1202-1207, 1998
Non-Patent Document 9
Hum. Immunol., 60: 10-19, 1999
Non-Patent Document 10
Hum. Immunol., 61: 357-365, 1999
Non-Patent Document 11
Annu. Rev. Immunol., 15, 535-562, 1997
Non-Patent Document 12
J. Exp. Med., 182, 633-638, 1995
Non-Patent Document 13
J. Exp. Med., 180: 1097, 1994
Non-Patent Document 14
Proc. Natl. Acad. Sci. USA, 88: 7518, 1991
Non-Patent Document 15
Proc. Natl. Acad. Sci. USA, 89: 6506, 1992
Non-Patent Document 16
J. Exp. Med., 176: 269, 1992
Non-Patent Document 17
J. Exp. Med., 179: 1285-1295, 1994
Non-Patent Document 18
J. Exp. Med., 186: 109, 1997
Non-Patent Document 19
J. Immunol., 161: 3271-3281, 1998
Non-Patent Document 20
Proc. Natl. Acad. Sci. USA, 95: 5690-5693, 1998
Non-Patent Document 21
Hum. Immunol., 61: 357-365, 1999
Non-Patent Document 22
Kawano, T. et al., Science 278: 1626, 1997
Non-Patent Document 23
Porcelli, S. et al. J Exp Med 178: 1-16, 1993
Non-Patent Document 24
Svennerholm, L. et al., Biochem. Biophys. Acta, 280, 626 (1972)
Non-Patent Document 25
Karlsson, K.-A. et al., Biochim. Biophys. Acta, 316,317 (1973)
Non-Patent Document 26
Morita, M. et al., J. Med. Chem., 38, 2176 (1995)
Non-Patent Document 27
Motoki, K. et al., Biol. Pharm. Bull., 18, 1487 (1995)
Non-Patent Document 28
Motoki, K. et al., Bioorg. Med. Chem. Lett., 5, 2413 (1995)
Non-Patent Document 29
Kobayashi, E. et al., Oncology Res., 7, 529 (1995)
Non-Patent Document 30
Motoki, K. et al., the report in the Annual Meeting of the Japanese Cancer Association, 523 (1996)
Non-Patent Document 31
Motoki, K. et al., Biol. Pharm. Bull., 19,952(1996)
Non-Patent Document 32
Eberl, G. et al., J. Immunol 162: 6410- 6419, 1999
Non-Patent Document 33
J. Exp. Med. 192: 741-753, 2000
Non-Patent Document 34
J. Exp. Med. 192: 921-930, 2000
Non-Patent Document 35
Toder, R. et al., Chromosome Res. 9: 431-435, 2001

### Disclosure of the Invention

An object of the present invention is to provide a therapeutic agent for inhibiting hepatitis C viruses, which comprises α-glycosylceramide.

As mentioned above, the effects of KRN7000 to inhibit HCV infections in humans had been problematic. However, the present inventors had examined whether or not KRN7000 had the effect of inhibiting HCV infections in the circumstances where development of a novel HCV inhibitor had been desired.

In general, chimpanzees infected with HCV are mainly used as test animal models for examining the anti-HCV effects of drugs because of the following reasons. That is, mice are not infected with HCV as with the case of HBV, and preparation of transgenic mouse models of HBV is difficult.

In the process of testing the drug efficacy of KRN7000 by utilizing HCV-infected chimpanzees, the present inventors have discovered that KRN7000 had the effects of inhibiting HCV. This has led to the completion of the present invention.

The inhibitor of hepatitis C viruses of the present invention comprises, as an active ingredient, a compound represented by formula (I) or a salt or solvate thereof: 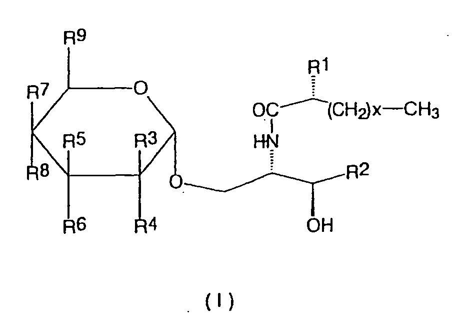 wherein
R¹ represents H or OH;
X is an integer between 7 and 27;
R² is any of substituents (a) to (e) below (wherein Y is an integer between 5 and 17):
   (a) -CH₂(CH₂)_{Y}CH₃;
   (b) -CH(OH)(CH₂)_{Y}CH₃;
   (c) -CH(OH)(CH₂)_{Y}CH(CH₃)₂;
   (d) -CH=CH(CH₂)_{Y}CH₃; or
   (e) -CH(OH)(CH₂)_{Y}CH(CH₃)CH₂CH₃; and
R³ to R⁹ are independently a substituent defined by any of i) to v) below:
   i) when R³, R⁶, and R⁸ independently represent H, R⁴ is H, OH, NH₂, NHCOCH₃, or a substituent defined by any of (A) to (D) below: 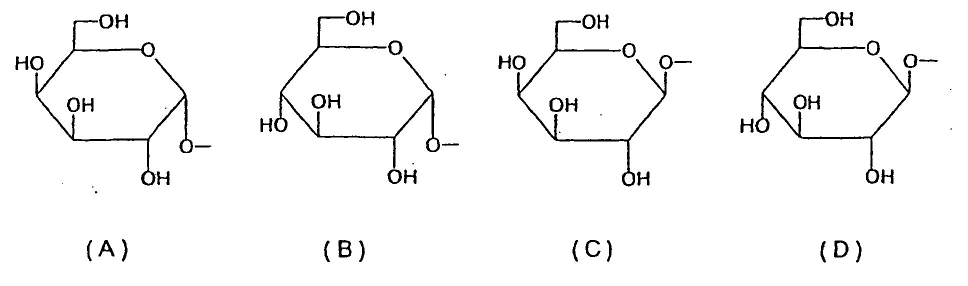
      R⁵ is OH or a substituent defined by (E) or (F) below: 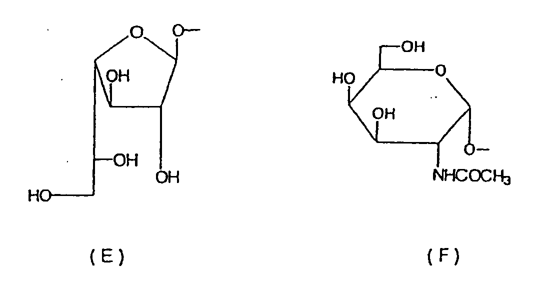
      R⁷ is OH or a substituent defined by any of (A) to (D) below: 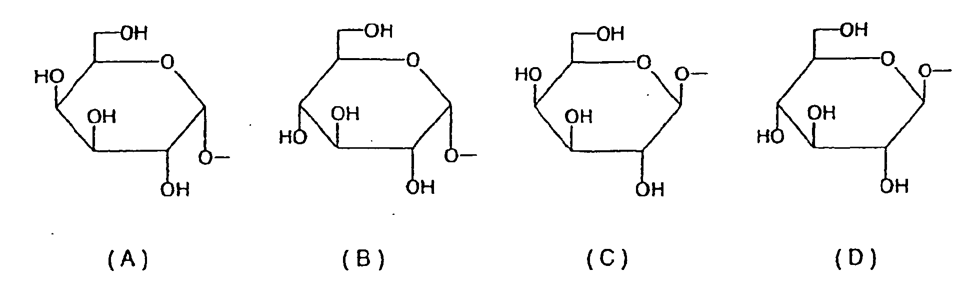
      R⁹ is H, CH₃, CH₂OH, or a substituent defined by any of (A') to (D') below: 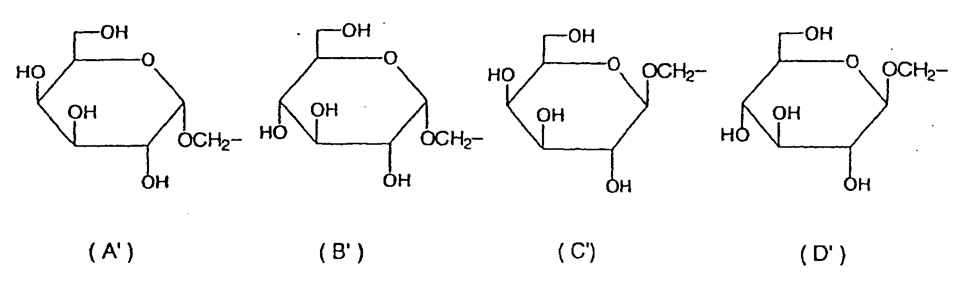 or
   ii) when R³, R⁶, and R⁷ are independently H, R⁴ is H, OH, NH₂, NHCOCH₃ or a substituent defined by any of (A) to (D) below: 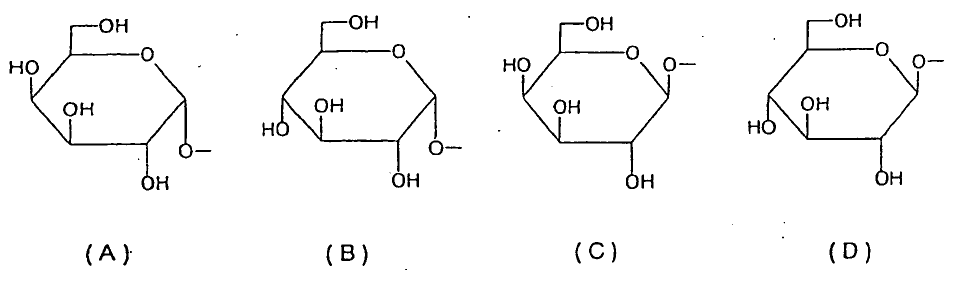
      R⁵ is OH or a substituent defined by (E) or (F) below: 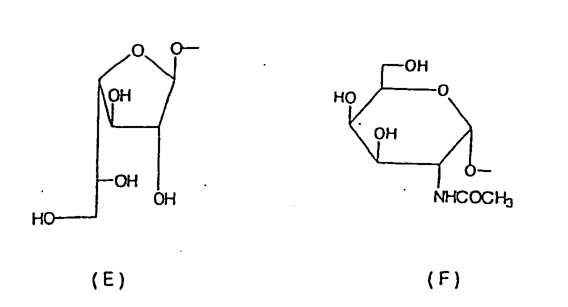
      R⁸ is OH or a substituent defined by any of (A) to (D) below: 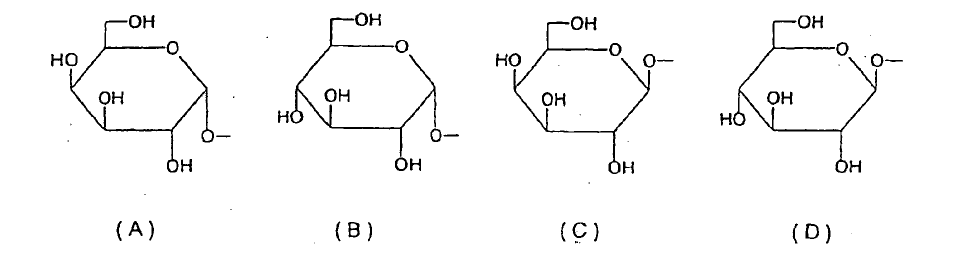 and
      R⁹ is H, CH₃, CH₂OH or a substituent defined by any of (A') to (D') below:

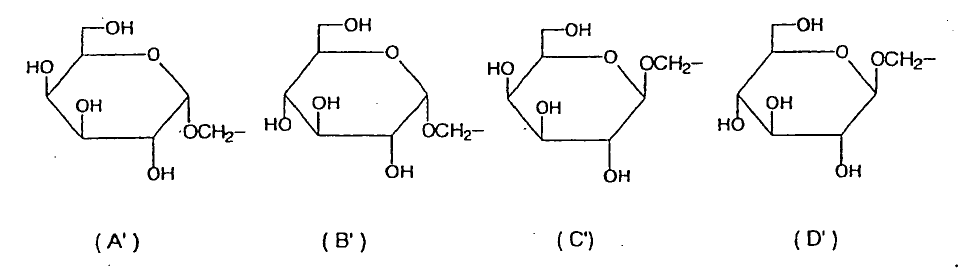

The method for synthesizing the compounds shown above may be implemented with reference to WO 98/44928.

More specifically, the present invention is as described below.
(1) An inhibitor of hepatitis C viruses comprising, as an active ingredient, a compound represented by formula (I) or a salt or solvate thereof.
(2) The inhibitor of hepatitis C viruses according to (1), wherein the hepatitis C virus is genotype 1.
(3) A therapeutic agent for hepatitis C comprising, as an active ingredient, the compound represented by formula (I) according to (1) or a salt or solvate thereof.
(4) The therapeutic agent for hepatitis C according to (3), wherein hepatitis C is chronic hepatitis C.
(5) The therapeutic agent for hepatitis C according to (3), wherein hepatitis C is acute hepatitis C.
(6) An agent for improving liver functions adversely affected due to hepatitis C comprising, as an active ingredient, the compound represented by formula (I) according to (1) or a salt or solvate thereof.
(7) The agent according to any of (1) to (6), wherein, in a compound represented by formula (I), R³ and R⁶ are independently H, R⁴ is OH or a substituent defined by any of (A) to (D), R⁵ is OH or a substituent defined by (E) or (F), R⁷ and R⁸ are independently H or OH, provided that R⁷ and R⁸ do not simultaneously represent the same group, and R⁹ represents CH₂OH, CH₃, H, or a substituent defined by any of (A') to (D').
(8) The agent according to any of (1) to (6), wherein, in a compound represented by formula (I), X is an integer between 21 and 25 and R² is a substituent (b) (wherein Y is an integer between 11 and 15).
(9) The agent according to any of (1) to (6), wherein, in a compound represented by formula (I), X is an integer between 9 and 13 and R² is a substituent (a) (wherein Y is an integer between 11 and 15).
(10) The agent according to any of (1) to (6), wherein a compound represented by formula (I) is selected from the group consisting of:
   (2S,3S,4R)-1-(α-D-galactopyranosyloxy)-2-hexacosanoylamino-3,4-octadecanediol;
   (2S,3R)-1-(α-D-galactopyranosyloxy)-2-tetradecanoylamino-3-octadecanol;
   (2S,3R)-1-(α-D-glucopyranosyloxy)-2-tetradecanoylamino-3-octadecanol;
   (2S,3R)-1-(6'-deoxy-α-D-galactopyranosyloxy)-2-tetradecanoylamino-3-octadecanol;
   (2S,3R)-1-(β-L-arabinopyranosyloxy)-2-tetradecanoylamino-3-octadecanol;
   O-α-D-galactopyranosyl-(1→6)-O-α-D-galactopyranosyl-(1→1)-(2S,3S,4R)-2-amino-N-hexacosanoyl-1,3,4-octadecanetriol;
   O-α-D-galactopyranosyl-(1→6)-O-α-D-glucopyranosyl-(1→1)-(2S,3S,4R)-2-amino-N-hexacosanoyl-1,3,4-octadecanetriol;
   O-α-D-galactopyranosyl-(1→2)-O-α-D-galactopyranosyl-(1→1)-(2S,3S,4R)-2-amino-N-[(R)-2-hydroxytetracosanoyl]-1,3,4-octadecanetriol;
   O-β-D-galactofuranosyl-(1→3)-O-α-D-galactopyranosyl-(1→1)-(2S,3S,4R)-2-amino-N-[(R)-2-hydroxytetracosanoyl]-1,3,4-octadecanetriol; and
   O-(N-acetyl-2-amino-2-deoxy-α-D-galactopyranosyl-(1→3)-O-[α-D-glucopyranosyl-(1→2)]-O-α-D-galactopyranosyl-(1→1)-(2S,3S,4R)-2-amino-N-[(R)-2-hydroxytetracosanoyl]-1,3,4-octadecanetriol.
(11) The agent according to any of (1) to (6), wherein a compound represented by formula (I) is selected from the group consisting of:
   (2S,3S,4R)-1-(α-D-galactopyranosyloxy)-2-hexacosanoylamino-3,4-octadecanediol;
   O-α-D-galactopyranosyl-(1→6)-O-α-D-galactopyranosyl-(1→1)-(2S,3S,4R)-2-amino-N-hexacosanoyl-1,3,4-octadecanetriol;
   O-α-D-galactopyranosyl-(1→6)-O-α-D-glucopyranosyl-(1→1)-(2S,3S,4R)-2-amino-N-hexacosanoyl-1,3,4-octadecanetriol;
   O-α-D-galactopyranosyl-(1→2)-O-α-D-galactopyranosyl-(1→1)-(2S,3S,4R)-2-amino-N-[(R)-2-hydroxytetracosanoyl]-1,3,4-octadecanetriol;
   O-β-D-galactofuranosyl-(1→3)-O-α-D-galactopyranosyl-(1→1)-(2S,3S,4R)-2-amino-N-[(R)-2-hydroxytetracosanoyl]-1,3,4-octadecanetriol; and
   O-(N-acetyl-2-amino-2-deoxy-α-D-galactopyranosyl-(1→3)-O-[α-D-glucopyranosyl-(1→2)]-O-α-D-galactopyranosyl-(1→1)-(2S,3S,4R)-2-amino-N-[(R)-2-hydroxytetracosanoyl]-1,3,4-octadecanetriol.
(12) The agent according to any of (1) to (6), wherein a compound represented by formula (I) is (2S,3S,4R)-1-(α-D-galactopyranosyloxy)-2-hexacosanoylamino-3,4-octadecanediol.

The present invention relates to an inhibitor of hepatitis C viruses comprising, as an active ingredient, the compound represented by formula (I) according to the present invention.

### (1) A compound represented by formula (I)

In the compound represented by formula (I), X in the ceramide portion is preferably an integer between 11 and 25.

Y represented by R² is preferably an integer between 9 and 17, and more preferably an integer between 11 and 15.

Examples of preferable X and R² combinations in the ceramide portion in formula (I) are a compound wherein X is an integer between 21 and 25 and R² is a substituent (b) (wherein Y is an integer between 11 and 15) and a compound wherein X is an integer between 9 and 13 and R² is a substituent (a) (wherein Y is an integer between 11 and 15).

An example of a preferable R³ to R⁹ combination in the sugar portion in formula (I) is a compound wherein R³ and R⁶ are independently H, R⁴ is OH or a substituent defined by any of (A) to (D), R⁵ is OH or a substituent defined by (E) or (F), R⁷ and R⁸ are independently H or OH, provided that R⁷ and R⁸ do not simultaneously represent the same group, and R⁹ is CH₂OH, CH₃, H, or a substituent defined by any of (A') to (D').

Examples of more preferable combinations thereof are a compound wherein R³ and R⁶ are independently H, R⁴ and R⁵ are independently OH, R⁷ and R⁸ are independently H or OH, provided that R⁷ and R⁸ do not simultaneously represent the same group, and R⁹ is CH₂OH or a substituent defined by any of (A') to (D') and a compound wherein R³, R⁶, and R⁸ are independently H, R⁴, R⁵, and R⁷ are independently OH, and R⁹ is CH₂OH.

Examples of preferable compounds represented by formula (I) include:
a compound wherein
   X is an integer between 21 and 25,
   R² is a substituent (b) (wherein Y is an integer between 11 and 15),
   R³ and R⁶ are independently H,
   R⁴ is OH or any of groups (A) to (D),
   R⁵ is OH or group (E) or (F),
   R⁷ and R⁸ are independently H or OH, provided that R⁷ and R⁸ do not simultaneously represent the same group, and
   R⁹ is CH₂OH or any of groups (A') to (D');
a compound wherein
   X is an integer between 9 and 13,
   R² is a substituent (a) (wherein Y is an integer between 11 and 15),
   R³ and R⁶ are independently H,
   R⁴ and R⁵ are independently OH,
   R⁷ and R⁸ are independently H or OH, provided that R⁷ and R⁸ do not simultaneously represent the same group, and
   R⁹ is H, CH₃, or CH₂OH;
a compound wherein
   X is an integer between 21 and 25,
   R² is a substituent (b) (wherein Y is an integer between 11 and 15),
   R³ and R⁶ are independently H,
   R⁴ and R⁵ are independently OH,
   R⁷ and R⁸ are independently H or OH, provided that R⁷ and R⁸ do not simultaneously represent the same group, and
   R⁹ is CH₂OH or any of groups (A') to (D'); and
a compound wherein
   X is an integer between 21 and 25,
   R² is a substituent (b) (wherein Y is an integer between 11 and 15),
   R³, R⁶, and R⁸ are independently H,
   R⁴, R⁵, and R⁷ are independently OH, and
   R⁹ is CH₂OH.
Examples of groups of compounds preferably used as active ingredients of agents according to the present invention include:
(2S,3S,4R)-1-(α-D-galactopyranosyloxy)-2-hexacosanoylamino-3,4-octadecanediol (KRN7000);
(2S,3R)-1-(α-D-galactopyranosyloxy)-2-tetradecanoylamino-3-octadecanol (AGL-517);
(2S,3R)-1-(α-D-glucopyranosyloxy)-2-tetradecanoylamino-3-octadecanol (AGL-563);
(2S,3R)-1-(6'-deoxy-α-D-galactopyranosyloxy)-2-tetradecanoylamino-3-octadecanol (AGL-571);
(2S,3R)-1-(β-L-arabinopyranosyloxy)-2-tetradecanoylamino-3-octadecanol (AGL-577);
O-α-D-galactopyranosyl-(1→6)-O-α-D-galactopyranosyl-(1→1)-(2S,3S,4R)-2-amino-N-hexacosanoyl-1,3,4-octadecanetriol (AGL-586);
O-α-D-galactopyranosyl-(1→6)-O-α-D-glucopyranosyl-(1→1)-(2S,3S,4R)-2-amino-N-hexacosanoyl-1,3,4-octadecanetriol (AGL-584);
O-α-D-galactopyranosyl-(1→2)-O-α-D-galactopyranosyl-(1→1)-(2S,3S,4R)-2-amino-N-[(R)-2-hydroxytetracosanoyl]-1,3,4-octadecanetriol (S1140B-9);
O-β-D-galactofuranosyl-(1→3)-O-α-D-galactopyranosyl-(1→1)-(2S,3S,4R)-2-amino-N-[(R)-2-hydroxytetracosanoyl]-1,3,4-octadecanetriol (719-7); and
O-(N-acetyl-2-amino-2-deoxy-α-D-galactopyranosyl-(1→3)-O-[α-D-glucopyranosyl-(1→2)]-O-α-D-galactopyranosyl-(1→1)-(2S,3S,4R)-2-amino-N-[(R)-2-hydroxytetracosanoyl]-1,3,4-octadecanetriol (STL-8).

Actions of these compounds for promoting the growth of NKT cells have been already verified (WO 98/44928).

A compound that is particularly preferably used as an active ingredient of the agent according to the present invention is (2S,3S,4R)-1-(α-D-galactopyranosyloxy)-2-hexacosanoylamino-3,4-octadecanediol (KRN7000).

A compound represented by formula (I) can be a pharmaceutically acceptable nontoxic salt. Examples of salts of the compound represented by formula (I) include acid addition salts, for example, salts with inorganic acids, such as hydrochloric acid, sulfuric acid, nitric acid, or phosphoric acid and salts with organic acids, such as acetic acid, propionic acid, maleic acid, oleic acid, palmitin acid, citric acid, succinic acid, tartaric acid, fumaric acid, glutamic acid, pantothenic acid, lauryl sulfonic acid, methanesulfonic acid, or phthalic acid.

A compound represented by formula (I) can be a solvate (e.g., a hydrate).

A compound represented by formula (I) can be produced by any process for synthesizing α-glycosylceramide.

A ceramide portion is first synthesized from D-lyxose, and sugar is then introduced into this ceramide portion. Thus, a compound represented by formula (I) can be prepared. References can be made to, for example, WO 93/5055, WO 94/2168, WO 94/9020, and WO 94/24142 concerning common processes for synthesizing α-glycosylceramide.

A compound represented by formula (I) can be isolated and purified from a naturally-occurring substance (e.g., an organism) by column chromatography or other means.

The agent according to the present invention inhibits the growth of hepatitis C viruses. Thus, it can be used as a therapeutic agent for hepatitis C. The agent according to the present invention can be used as a therapeutic agent for both chronic and acute hepatitis C. Infectious hepatitis C virus diseases or hepatitis C can be treated by administering the agent of the present invention to patients who had been infected with hepatitis C viruses or patients who had been infected with hepatitis C and then developed symptoms of hepatitis. Continuous administration of the agent of the present invention to patients for a given period of time can completely eliminate hepatitis C viruses. Further, the agent of the present invention can alleviate a variety of hepatitis C symptoms resulting from hepatitis C virus infections. Continuous administration of the agent of the present invention to patients for a given period of time can completely eliminate hepatitis C. In cases where the viruses are not completely eliminated, the agent can inhibit virus growth within a patient, suppress symptoms of hepatitis C virus infections, enhance liver functions, and keep the disease state from advancing to hepatic cirrhosis or hepatocarcinoma.

A compound represented by formula (I) or a salt or solvate thereof can be prepared in a suitable dosage form in accordance with method of treatment, route of administration, and purpose of administration. Specific examples of dosage forms include preparations such as parenteral injections, suspensions, emulsifiers, ointments, creams, tablets, capsules, granules, powders, pills, fine grains, troches, agents for rectal administration, oleaginous suppositories, and water-soluble suppositories.

These preparations can be produced by a conventional technique using, for example, pharmaceutically acceptable carriers described below. Examples of carriers are: excipients such as solvents (e.g., water and physiological saline), extenders, and fillers (e.g., lactose, starch, crystalline cellulose, mannitol, maltose, calcium hydrogen phosphate, soft silicic acid anhydride, and calcium carbonate); adjuvants such as solubilizers (e.g., ethanol and polysorbates), binders (e.g., starch, polyvinyl pyrrolidone, hydroxypropylcellulose, ethylcellulose, carboxymethylcellulose, and gum Arabic), disintegrators (e.g., starch and carboxymethylcellulose calcium), lubricants (e.g., magnesium stearate, talc, and hardened oil), stabilizers (e.g., lactose, mannitol, maltose, polysorbates, macrogols, and polyoxyethylene hardened castor oil), isotonizing agents, wetting agents, lubricants, dispersants, buffers, and solubilizers; and additives, such as antioxidants, preservatives, flavoring agents, soothing agents, stabilizers, colorants, and sweetening agents.

These preparations can additionally comprise glycerin, dimethylacetamide, sodium lactate (70%), surfactants, or basic substances (e.g., ethylenediamine, ethanolamine, sodium carbonate, arginine, meglumine, or tris-aminomethane) according to need.

In the present invention, a compound represented by formula (I) can be administered by any route in accordance with a purpose of administration. More specifically, this compound can be administered intraperitoneally, subcutaneously, intravascularly into vein or arteries, or topically by injections to animals. This compound can be administered intravenously, intraarterially, topically by injections, intraperitoneally, intrathoracically, subcutaneously, intramuscularly, sublingually, percutaneously, or rectally to humans. Intravenous or subcutaneous administration is the most preferable route of administration.

Each of the active ingredients in the therapeutic agent of the present invention can be continuously or intermittently administered in accordance with conditions. A specific dose varies in accordance with the route of administration and a variety of conditions of patients, for example, the age, body weight, sex, and sensitivity of the patient, the duration of administration, or a type of agent used in combination therewith. In general, the dose of a compound represented by formula (I) is preferably approximately 0.001 mg to 10 mg, preferably 0.05 mg to 2 mg, and more preferably 0.01 mg to 1 mg, per adult per day in the case of intravenous administration. A compound represented by formula (I) is preferably in the form of a lyophilized preparation. Preferably, this preparation is dissolved in distilled water for injection or the like immediately before administration and then administered. Administration is carried out for a given period of time, for example, every several days to every several months. Administration is preferably continued for a given period of time.

The effects of inhibiting hepatitis C viruses can be evaluated by periodical monitoring of the occurrence of hepatitis C virus infections and vital titers thereof. The titer of hepatitis C viruses can be monitored by assaying the RNA level of hepatitis C viruses by RT-PCR or other means. Decreased or eliminated hepatitis C viruses leads to healing of hepatitis and enhancement of liver functions that had been adversely affected while the patient had been infected with hepatitis C viruses. Enhanced liver functions can be monitored by measuring levels of ALT, AST, and LDH in serums.

The present invention further relates to: a process for inhibiting hepatitis C viruses comprising administering the agent of the present invention to patients infected with hepatitis C viruses; a process for treating hepatitis C comprising administering the agent of the present invention to patients infected with hepatitis C viruses; and a process for enhancing liver functions that had been adversely affected due to hepatitis C virus infections comprising administering the agent of the present invention to patients having deteriorated liver functions due to hepatitis C virus infections. Furthermore, the present invention relates to the use of a compound represented by formula (I) in an inhibitor of hepatitis C viruses or a therapeutic agent for hepatitis C and in the production of an agent for enhancing liver functions adversely affected due to hepatitis C virus infections. Also, the present invention includes the use of a compound represented by formula (I) in combination with interferon or other antiviral agents, such as ribavirin (RBV).

This description includes part or all of the contents as disclosed in the description and/or drawings of Japanese Patent Application No. 2002-275466, which is a priority document of the present application.

### Brief Description of the Drawings

Fig. 1 shows changes in the total leukocyte count, the neutrophil count, and the lymphocyte count in bloods of 2 chimpanzees infected with hepatitis C viruses to which KR7000 had been administered over a period of time.
Fig. 2A shows the results of assaying NKT cells of chimpanzees infected with hepatitis C viruses before KR7000 administration by FACS.
Fig. 2B shows changes in the ratio of the NKT cell count to the T cell count of 2 chimpanzees infected with hepatitis C viruses to which KR7000 had been administered over a period of time.
Fig. 3 shows changes in levels of ALT, AST, and LDH in serums of 2 chimpanzees infected with hepatitis C viruses to which KR7000 had been administered over a period of time.
Fig. 4 shows the increase and decrease of hepatitis C viruses of 2 chimpanzees infected with hepatitis C viruses to which KR7000 had been administered.
Fig. 5 schematically shows the path of synthesis of a representative α-glycosylceramide compound, KRN7000, that is used in the present invention.
Fig. 6 schematically shows the path of synthesis subsequent to that shown in Fig. 5.

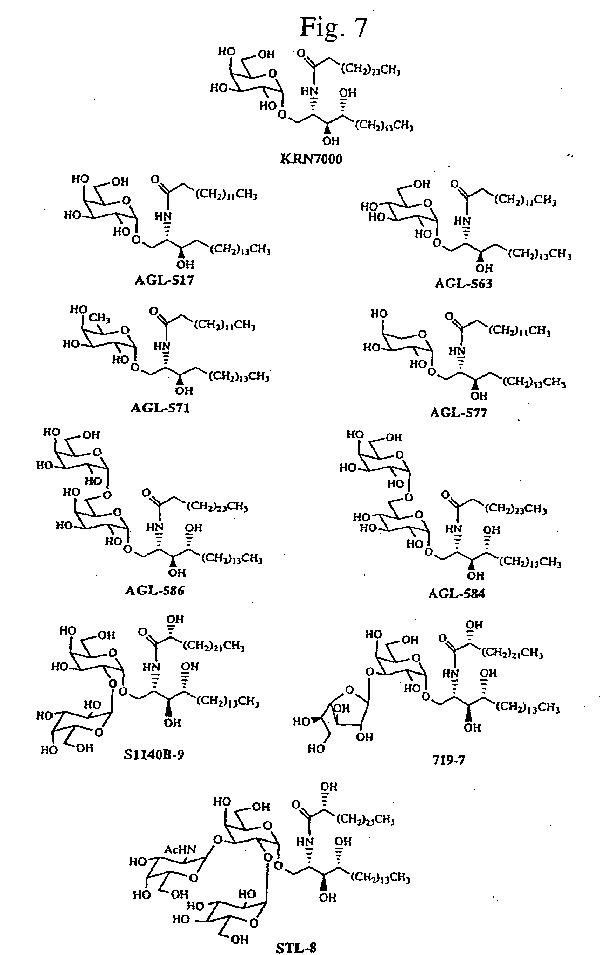

Fig. 7 shows chemical formulae representing compounds prepared in Examples 1 to 3.

### Best Modes for Carrying out the Invention

The present invention is hereafter described in more detail with reference to the following examples, although the technical scope of the present invention is not limited to these examples.

### Synthesis, isolation, and purification of compounds

### [Example 1] Synthesis of (2S,3S,4R)-1-(α-D-galactopyranosyloxy)-2-hexacosanoylamino-3,4-octadecanediol (KRN7000)

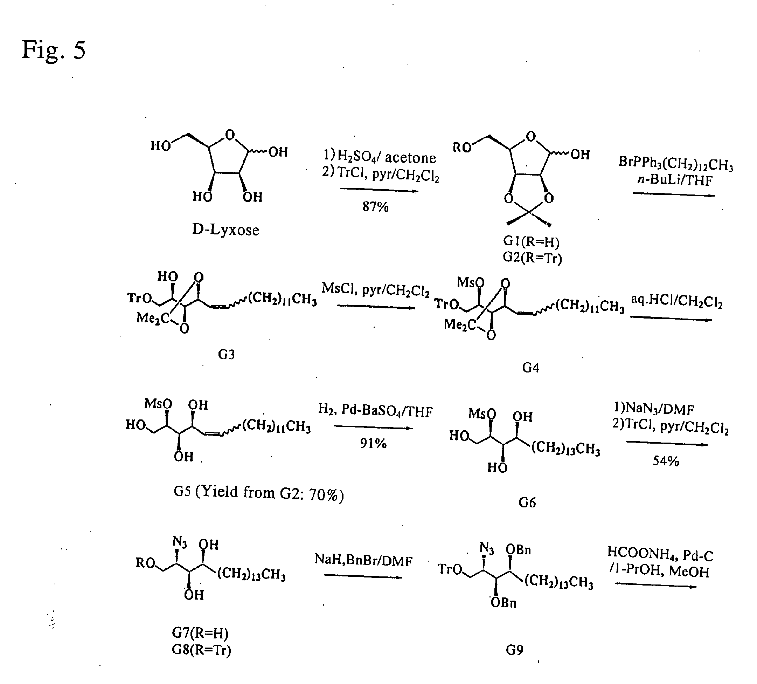

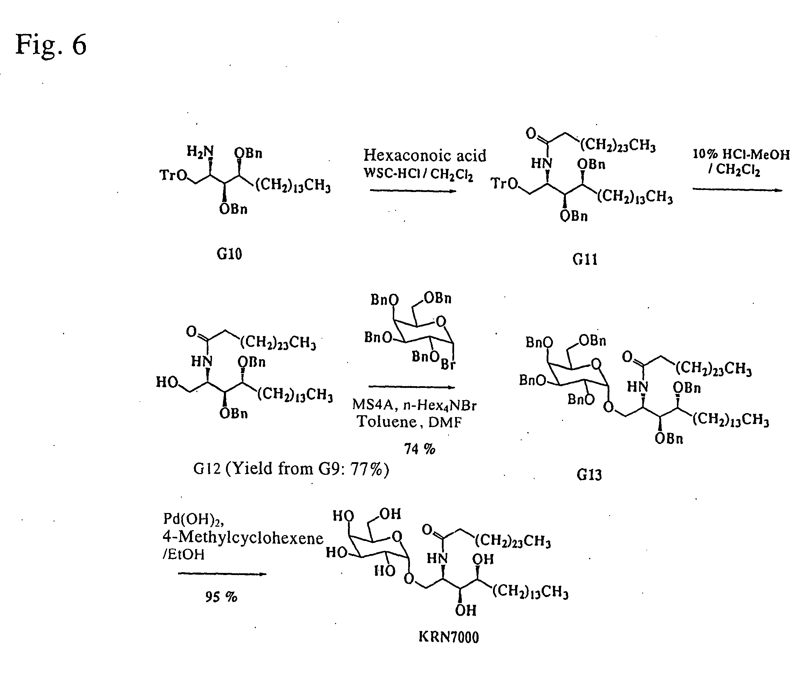

Processes of synthesis are as shown in Figs. 5 and 6. In the reaction processes shown in Fig. 5, pyr denotes pyridine, BrPPh₃(CH₂)₁₂CH₃ denotes tridecane triphenylphosphonium bromide, n-BuLi denotes n-butyllithium, MsCl denotes methanesulfonyl chloride, BnBr denotes benzyl bromide, and 1-PrOH denotes propyl alcohol. In the reaction processes shown in Fig. 6, WSC-HCl denotes 1-ethyl-3-(3'-dimethylaminopropyl)carbodiimide hydrochloride, MS4A denotes Molecular Sieves 4A, and Hex4NBr denotes tetrahexylammonium bromide.

### (1) Synthesis of Compound G1

An acetone solution (3.0 l) and sulfuric acid (0.5 ml) dried over calcium chloride were added to D-lyxose (200 g, 1.33 mol), and the mixture was agitated at room temperature for 18 hours. The reaction solution was neutralized with the addition of Molecular Sieves 4A powder (100 g), the neutralized solution was filtered through celite, and the residue was washed with acetone. The filtrate and a washing solution were combined and concentrated under reduced pressure, thereby obtaining a crude product, Compound G1 (yield: 240 g, 95%). This product was used in the subsequent process without further purification. An analyte was purified by silica gel chromatography using hexane/acetone (9/1) as an eluent.
mp: 76-78°C
FDMS: m/z 191(M+1)⁺
¹H-NMR (500 MHz, CDCl₃) δ 5.45 (1H, d, J = 1.8 Hz), 4.83 (1H, dd, J = 3.7, 5.5 Hz), 4.64 (1H, d, J = 6.1 Hz), 4.27-4.30 (1H, m), 3.90-3.99 (2H, m), 1.48 (3H, s), 1.32 (3H, s)

### (2) Synthesis of Compound G2

Pyridine (10 ml) and triphenylmethyl chloride (39.0 g) were added to 168 ml of a methylene chloride solution containing Compound G1 (239 g, approximately 1.26 mmol), and the mixture was agitated at 32°C for 4 hours. Ethanol (8 ml) was added dropwise thereto, and the mixture was agitated at room temperature for 2 hours. After being washed with a saturated aqueous solution of ammonium chloride, a saturated aqueous solution of sodium bicarbonate, and a saline solution, the resultant was concentrated under reduced pressure. The residue was dissolved in ethyl acetate and cooled to 0°C for crystallization (yield: 501 g, 87% yielded from D-lyxose).
mp: 174-176°C
FDMS: m/z 432M⁺
¹H-NMR (500 MHz, CDCl₃) δ 7.21-7.49 (15H, m), 5.38 (1H, d, J = 2.4 Hz), 4.75 (1H, dd, J = 3.7, 6.1 Hz), 4.59 (1H, d, J = 6.1 Hz), 4.31-4.35 (1H, m), 3.43 (1H, dd, J = 4.9, 9.8 Hz), 3.39 (1H, dd, J = 6.7, 9.8 Hz), 1.29 (3H, s), 1.28 (3H, s)

### (3) Synthesis of Compound G3

1-Bromotridecane and triphenylphosphine were heated at 140°C for 4.5 hours to obtain tridecane triphenylphosphonium bromide (962 g, 1.16 mol). The resultant was then added to THF to prepare 1,500 ml of a solution. A solution of n-butyllithium in 2.5M hexane (462 ml, 366 mmol) was added dropwise thereto at 0°C under argon gas. After the dropwise addition, the mixture was agitated for 15 minutes, and 450 ml of a THF solution containing Compound G2 (250 g, 579 mmol) was added dropwise thereto. The mixture was agitated for 18 hours while gradually raising the temperature thereof to room temperature. The reaction solution was concentrated under reduced pressure, 1,000 ml of a hexane/methanol/water mixture (10/7/3) was added to the residue, and the resultant was washed with a saturated aqueous solution of ammonium chloride. The aqueous phase was extracted with 500 ml of hexane, and all the organic layers were combined, followed by drying over anhydrous magnesium sulfate and concentration under reduced pressure. Thus, a crude product, Compound G3, was obtained (yield: 339 g, 98%). This product was used in the subsequent process without further purification. An analyte was purified by silica gel chromatography using hexane/ethyl acetate (9/1) as an eluent.
FDMS: m/z 598M⁺
¹H-NMR (500 MHz, CDCl₃) δ 7.21-7.45 (15H, m), 5.48-5.59 (2H, m), 4.91 (0.7H, t, J = 7.3 Hz), 4.44 (0.3H, t, J = 7.3 Hz), 4.26 (0.3H, dd, J = 4.3, 7.3 Hz), 4.21 (0.7H, dd, J = 4.3, 6.7 Hz), 3.75 (0.7H, m), 3.69 (0.3H, m), 3.24 (0.3H, dd, J = 4.9, 9.8 Hz), 3.17 (0.7H, dd, J = 4.9, 9.8 Hz), 3.09-3.14[1H, (3.11, dd, J = 4.9, 9.2 Hz), H1bE overlapped], 1.75-2.03 (2H, m), 1.49 (3H, s), 1.39 and 1.38 (3H, each s), 1.21-1.34 (20H, m), 0.88 (3H, t, J = 6.7 Hz)

### (4) Synthesis of Compound G4

Pyridine (500 ml) was added to 1,500 ml of a methylene chloride solution containing Compound G3 (338 g, approximately 565 mmol), and methanesulfonyl chloride (49 ml, 633 mmol) was added dropwise thereto, followed by agitation at 31°C for 24 hours. Ethanol (40 ml) was added dropwise thereto, and the mixture was agitated at room temperature for 1 hour. After having been concentrated under reduced pressure, 1,000 ml of a hexane/methanol/water mixture (10/7/3) was added to the residue, and the resultant was separated. The aqueous phase was extracted three times with 200 ml of hexane each time, and all the organic phases were combined, followed by drying over anhydrous magnesium sulfate and concentration under reduced pressure. Thus, a crude product, Compound G4, was obtained (yield: 363 g, 95%). This product was used in the subsequent process without further purification. An analyte was purified by silica gel chromatography using hexane/ethyl acetate (9/1) as an eluent.
FDMS: m/z 676M⁺
¹H-NMR (500 MHz, CDCl₃) δ 7.21-7.47 (15H, m), 5.41 (0.7H, ddd, J = 5.5, 9.2, 11.0 Hz), 5.32 (0.7H, bt, J = 11.0 Hz), 5.22 (0.3H, bdd, J = 9.2, 15.0 Hz), 5.02 (0.3H, dt, Jₜ = 7.3 Hz, J_{d} = 15.0 Hz), 4.8 (0.7H, ddd, J = 3.1, 5.5, 7.9 Hz), 4.73 (0.7H, dd, J = 5.5, 9.8 Hz), 4.64-4.67 (0.3H, m), 4.61 (0.3H, dd, J = 5.5, 9.2 Hz), 4.48 (0.7H, dd, J = 5.5, 7.9 Hz), 4.22 (0.3H, dd, J = 5.5, 9.2 Hz), 3.55 (0.3H, dd, J = 2.4, 11.6 Hz), 3.45 (0.7H, dd, J = 3.2, 11.0 Hz), 3.06-3.12 [4H, (3.12, s), (3.11, s), (3.09, dd, J = 3.1, 11.0 Hz)], 1.66-1.82 (2H, m), 1.47 and 1.46 (3H, each s), 1.39 (3H, s), 1.13-1.35 (20H, m), 0.88 (3H, t, J = 6.8 Hz)

### (5) Synthesis of Compound G5

Methanol (350 ml) was added to 1,500 ml of a methylene chloride solution containing Compound G4 (362 g, approximately 536 mmol), concentrated hydrochloric acid (200 ml) was added dropwise thereto, and the mixture was agitated at room temperature for 5 hours. The reaction solution was neutralized with the addition of sodium bicarbonate, and the neutralized solution was filtered. The filtrate was concentrated under reduced pressure, ethyl acetate was added to the residue, and the resultant was washed with a saline solution. The aqueous phase was extracted with ethyl acetate, and all the organic phases were combined, followed by drying over anhydrous magnesium sulfate and concentration under reduced pressure. The residue was crystallized from hexane (yield: 161 g, 70% yielded from G2).
mp: 66-67°C
FDMS: m/z 377 (M-H₂O)⁺
¹H-NMR (500 MHz, CDCl₃+D₂O) δ 5.86 (0.3H, dt, Jₜ = 7.3 Hz, J_{d} = 14.7 Hz), 5.77 (0.7H, dt, Jₜ = 7.3, J_{d} = 10.4 Hz), 5.55 (0.3H, br. dd, J = 7.3, 14.7 Hz), 5.49 (0.7H, bt, J = 9.8 Hz), 4.91-4.97 (1H, m), 4.51 (0.7H, bt, J = 9.8 Hz), 4.11 (0.3H, bt, J = 7.3 Hz), 3.94-4.03 (2H, m), 3.67-3.73 [1H, (3.70, dd, J = 3.1, 6.7 Hz), (3.69, dd, J = 3.1, 7.3 Hz)], 3.20 and 3.19 (3H, each s), 2.05-2.22 (2H, m), 1.22-1.43 (20H, m), 0.88 (3H, t, J = 6.7 Hz)

### (6) Synthesis of Compound G6

5% Palladium-barium sulfate (16 g) was added to 780 ml of a THF solution containing Compound G5 (160 g, 405 mmol), the air in the reaction chamber was replaced with hydrogen gas, and the contents therein were agitated at room temperature for 20 hours. The reaction solution was filtered through celite and washed with a chloroform/methanol mixture (1/1). The filtrate and a washing solution were combined and concentrated under reduced pressure. The residue was crystallized from ethyl acetate (yield: 146 g, 91%).
[α]²³_{D}+12° (c 1, CHCl₃/MeOH = 1/1)
mp: 124-126°C
FDMS: m/z 397 (M+1)⁺
¹H-NMR (500 MHz, CDC₃l/CD₃OD = 1/1) α 4.93-4.96 (1H, m, H2), 3.91 (1H, dd, J = 6.7, 12.2 Hz), 3.85 (1H, dd, J = 4.9, 12.2 Hz), 3.54-3.60 (1H, m), 3.50 (1H, dd, J = 1.8, 8.5 Hz), 3.19 (3H, s), 1.75-1.83 (1H, m), 1.53-1.62 (1H, m), 1.21-1.45 (24H, m), 0.89 (3H, t, J = 6.7 Hz)

### (7) Synthesis of Compound G7

Sodium azide (47 g, 730 mmol) was added to 1,000 ml of a DMF solution containing Compound G6 (145 g, 365 mmol), and the mixture was agitated at 95°C for 4 hours. The reaction solution was concentrated, ethyl acetate (450 ml) was added to the residue, and the resultant was rinsed. The aqueous phase was extracted again with ethyl acetate. All the organic phases were combined, washed with a saline solution, dried over anhydrous magnesium sulfate, and concentrated under reduced pressure. Thus, a crude product, Compound G7, was obtained (yield: 122 g, 97%). This product was used in the subsequent process without further purification (yield: 126 g, 95%). An analyte was purified by silica gel chromatography using hexane/ethyl acetate (9/1) as an eluent.
[α]²³_{D}+16.5° (c 0.5, CHCl₃/MeOH = 1/1)
mp: 92-93°C .
FDMS: m/z 344 (M+1)⁺
¹H-NMR (500 MHz, CD₃OD) δ 3.91 (1H, dd, J = 3.7, 11.6 Hz), 3.75 (1H, dd, J = 7.9, 11.6 Hz), 3.49-3.61 (3H, m), 1.50-1.71 (2H, m), 1.22-1.46 (24H, m), 0.90 (3H, t, J = 6.7 Hz)

### (8) Synthesis of Compound G8

Pyridine (250 ml) and triphenylmethyl chloride (124 g, 445 mmol) were added to 750 ml of a methylene chloride solution containing Compound G7 (121 g, approximately 352 mmol), and the mixture was agitated at room temperature for 16 hours. Ethanol (30 ml) was added dropwise thereto, and the mixture was agitated at room temperature for 30 minutes. Thereafter, the resultant was washed with a saturated aqueous solution of sodium bicarbonate, a saturated aqueous solution of ammonium chloride, and a saline solution, and the resultant was dried over anhydrous magnesium sulfate, followed by concentration under reduced pressure. The residue was purified by silica gel chromatography using hexane/ethyl acetate (10/1) as an eluent (yield: 34.4 g, 52% yielded from G6).
[α]²⁴_{D}+11.9° (c 0.9, CHCl₃)
FDMS: m/z 585M⁺
¹H-NMR (500 MHz, CDCl₃+D₂O) δ 7.24-7.61 (15H, m), 3.62-3.66 (2H, m), 3.51-3.57 (2H, m), 3.42 (1H, dd, J = 6.0, 10.4 Hz), 1.23-1.56 (26H, m), 0.88 (3H, t, J = 6.7 Hz)

### (9) Synthesis of Compound G9

Sixty percent sodium hydride (5.5 g, approximately 138 mmol of NaH) was added to 300 ml of a DMF solution containing Compound G8 (33.5 g, 57.3 mmol), and the mixture was agitated at room temperature for 40 minutes. The reaction solution was cooled to 0°C and benzyl bromide (15 ml, 120 mmol) was added dropwise thereto. The mixture was agitated for 18 hours while gradually raising the temperature thereof to room temperature. Ice water (100 ml) was added to the reaction solution to terminate the reaction. Thereafter, the aqueous phase was extracted with ethyl acetate. The extract was washed three times with a saline solution each time, and all the organic phases were combined, followed by drying over anhydrous magnesium sulfate and concentration under reduced pressure. Thus, a crude product, Compound G9, was obtained (yield: 42.2 g, 96%). This product was used in the subsequent process without further purification. An analyte was purified by silica gel chromatography using hexane/ethyl acetate (100/1) as an eluent.
[α]²⁴_{D}+9.8° (c 1.0, CHCl₃)
FDMS: m/z 738 (M-N₂)⁺
¹H-NMR (500 MHz, CDCl₃) δ 7.07-7.48 (25H, m), 4.57 (1H, d, J = 11.6 Hz), 4.44 (1H, d, J = 11.6 Hz), 4.41 (2H, s), 3.73-3.79 (1H, m), 3.46-3.56 (2H, m), 3.37 (1H, dd, J = 8.6, 10.4 Hz), 1.20-1.64 (26H, m), 0.88 (3H, t, J = 6.7 Hz)

### (10) Syntheses of Compounds G10 and G11

Methanol (30 ml) was added to 250 ml of a 1-propanol solution containing Compound G9 (41.2 g, approximately 54 mmol), and 5% palladium-carbon (4.1 g) and ammonium formate (27.1 g, 4.3 mol) were further added thereto. The mixture was agitated at room temperature for 16 hours, diluted with ethyl acetate, and then filtered through celite. The filtrate was concentrated under reduced pressure, dissolved in ethyl acetate, and washed three times with a saturated aqueous solution of sodium bicarbonate and a saline solution each time. All the organic phases were combined, followed by drying over anhydrous magnesium sulfate and concentration under reduced pressure. Thus, a crude product, Compound G10, was obtained (yield: 38.9 g, 98%). The resulting Compound G10 was used in the subsequent process without further purification.

Hexacosanoic acid (22.4 g, 56.5 mmol) and WSC hydrochloride (12.6 g, 64.6 mmol) were added to 300 ml of a methylene chloride solution containing Compound G10, and the mixture was heated under reflux for 2 hours. The temperature of the mixture was cooled to room temperature, and the mixture was concentrated under reduced pressure. Ethyl acetate (500 ml) was added to the residue, and the resultant was washed with an aqueous solution of 0.5M hydrochloric acid, a saline solution, a saturated aqueous solution of sodium bicarbonate, and a saline solution. All the organic phases were combined, followed by drying over anhydrous magnesium sulfate and concentration under reduced pressure. Thus, a crude product, Compound G11, was obtained (yield: 53.2 g, 88%). The resulting Compound G11 was used in the subsequent process without further purification. An analyte was purified by silica gel chromatography using hexane/ethyl acetate (100/1) as an eluent.
[α]²⁴_{D}+5.3° (c 0.4, CHCl₃)
FDMS: m/z 1118M⁺
¹H-NMR (500 MHz, CDCl₃) δ 7.20-7.38 (25H, m), 5.57 (1H, d, J = 9.1 Hz), 4.80 (1H, d, J = 11.6 Hz), 4.48-4.50 (3H, m), 4.24-4.32 (1H, m), 3.83 (1H, dd, J = 3.0, 6.7 Hz), 3.43-3.51 (2H, m, H1a), 3.29 (1H, dd, J = 4.3, 9.8 Hz), 1.92 (2H, t, J = 7.3 Hz), 1.28-1.60 (72H, m), 0.88 (6H, t, J = 6.7 Hz)

### (11) Synthesis of Compound G12

Methanol (36 ml) was added to 180 ml of a methylene chloride solution containing Compound G11 (52.2 g, approximately 47 mmol), 3.0 ml of a solution of 10% hydrochloric acid in methanol was then added dropwise thereto, and the mixture was agitated at room temperature for 2 hours. The reaction solution was neutralized with 18 g of sodium bicarbonate powder and then filtered through celite. The residue was washed with methylene chloride. The filtrate and a washing solution were combined, the resultant was washed with a saline solution, and the organic phase was dried over anhydrous magnesium sulfate, followed by concentration under reduced pressure. The residue was dissolved in acetone with heating, cooled to 0°C, and purified by precipitation (yield: 38.6 g, 77% yielded from G9).
[α]²⁴_{D}-29.7° (c 0.7, CHCl₃)
mp: 75-76.5°C
FDMS: m/z 876M⁺
¹H-NMR (500 MHz, CDCl₃) δ 7.30-7.47 (10H, m), 6.03 (1H, d, J = 7.9 Hz), 4.72 (1H, d, J = 11.6 Hz), 4.66 (1H, d, J = 11.6 Hz), 4.61 (1H, d, J = 11.6 Hz), 4.45 (1H, d, J = 11.6 Hz), 4.12-4.17 (1H, m), 4.00 (1H, dt, Jₜ = 4.3, J_{d} = 7.3 Hz), 3.67-3.72 (2H, m), 3.61 (1H, ddd, J = 4.3, 8.6, 11.6 Hz), 1.94-2.05 (2H, m), 1.15-1.69 (72H, m), 0.88 (6H, t, J = 6.1 Hz)

### (12) Synthesis of Compound G13

1) 2,3,4,6-Tetra-O-benzyl-D-galactopyranosyl acetate (79.8 g) was dissolved in a mixture of toluene (160 ml) and isopropyl ether (520 ml), and the mixture was cooled to between -10°C and 0°C. An isopropyl ether solution containing 2.0 equivalents of HBr was added thereto (2.8 mmol/ml, approximately 100 ml). After the mixture was agitated at between -10°C and 0°C for approximately 90 minutes, an aqueous solution of 5% sodium bicarbonate was added to the reaction solution, and the excess amount of HBr was neutralized by agitation. The total amount thereof was transferred to a separatory funnel to fractionate the solution. Thereafter, the aqueous layer was discarded, and the residue was washed two times with an aqueous solution of 10% sodium chloride each time. The resultant was concentrated under reduced pressure to obtain syrup of 2,3,4,6-tetra-O-benzyl-α-D-galactopyranosyl bromide (GalBr).
2) DMF (140 ml) and 250 ml of a solution of GalBr (approximately 137 mmol) in toluene were added in that order to 420 ml of a solution containing Compound G12 (60.0 g, 68.6 mmol), tetrahexylammonium bromide (89.4 g, 206 mmol), and Molecular Sieves 4A (60 g) in toluene. The mixture was agitated at room temperature for 72 hours. Methanol (12 ml) was added to the reaction solution and the mixture was agitated for 2 hours. After the solution was filtered through celite, it was washed with a saturated aqueous solution of sodium bicarbonate and a saline solution, followed by drying over anhydrous magnesium sulfate and concentration under reduced pressure. Acetonitrile was added to the residue, and the resultant was agitated for 2 hours to obtain a precipitate. The resulting precipitate was dehydrated under reduced pressure to obtain dry powder. The resultant was purified by silica gel chromatography using hexane/ethyl acetate (8/1) as an eluent (yield: 70.9 g, 74%).
   [α]²⁴_{D}+18.8° (c 0.9, CHCl₃)
   mp: 74-75°C
   FDMS: m/z 1399 (M+1)⁺
   ¹H-NMR (500 MHz, CDCl₃) δ 7.21-7.37 (30H, m), 6.12 (1H, d, J = 9.0 Hz), 4.91 (1H, d, J = 11.6 Hz), 4.84 (1H, d, J = 3.7 Hz), 4.72-4.80 (4H, m), 4.35-4.65 (7H, m), 4.12-4.18 (1H, m), 3.99-4.05 (2H, m), 3.84-3.93 (4H, m), 3.73 (1H, dd, J = 3.7, 11.0 Hz), 3.47-3.51 (2H, m), 3.42 (1H, dd, J = 6.1, 9.1 Hz), 1.87-1.99 (2H, m), 1.18-1.70 (72H, m), 0.88 (6H, t, J = 7.4 Hz)

### (13) Synthesis of Compound, KRN7000

Compound G13 (60.0 g, 42.9 mmol) was added to ethanol (960 ml) to suspend it therein, and an ethanol suspension comprising 20% palladium hydroxide (6.0 g) was added thereto. A hydrogen source, i.e., 4-methylcyclohexene (120 ml, 93.5 mmol) was further added thereto, the mixture was heated under reflux for 4 hours, and the catalyst was removed by filtration. The residue was washed with heated ethanol. The filtrate was allowed to stand at room temperature to obtain a white precipitate. The resulting precipitate was filtered and then dehydrated under reduced pressure. The obtained powder was suspended in 3.5 1 of ethanol/water (92/8), dissolved therein by heating while agitating, and allowed to stand at room temperature for reprecipitation. The precipitate was filtered, and the cake obtained by filtration was dehydrated under reduced pressure to obtain white powder (yield: 35.0 g, 95%).
[α]²³_{D}+43.6° (c 1.0, pyridine)
mp: 189.5-190.5°C
negative FABMS: m/z 857 (M-H)⁻
IR (cm⁻¹, KBr) 3300, 2930, 2850, 1640, 1540, 1470, 1070
¹H-NMR (500 MHz, C₅D₅N) δ 8.47 (1H, d, J = 8.5 Hz), 5.58 (1H, d, J = 3.7 Hz), 5.27 (1H, m), 4.63-4.70 (2H, m), 4.56 (1H, m), 4.52 (1H, t, J = 6.1 Hz), 4.37-4.47 (4H, m), 4.33 (2H, m), 2.45 (2H, t, J = 7.3 Hz), 2.25-2.34 (1H, m), 1.87-1.97 (2H, m), 1.78-1.85 (2H, m), 1.62-1.72 (1H, m), 1.26-1.45 (66H, m), 0.88 (6H, t, J = 6.7 Hz)
¹³C-NMR (125 MHz, C₅D₅N) δ 173.2 (s), 101.5 (d), 76.7 (d), 73.0 (d), 72.5 (d), 71.6 (d), 71.0 (d), 70.3 (d), 68.7 (t), 62.7 (t), 51.4 (d), 36.8 (t), 34.4 (t), 32.1 (t), 30.4 (t), 30.2 (t), 30.03 (t), 30.00 (t), 29.93 (t), 29.87 (t), 29.81 (t), 29.76 (t), 29.6 (t), 26.5 (t), 26.4 (t), 22.9 (t), 14.3 (q)

### [Example 2] Isolation and purification of O-α-D-galactopyranosyl-(1→2)-O-α-D-galactopyranosyl-(1→1)-(2S,3S,4R)-2-amino-N-[(R)-2-hydroxytetracosanoyl]-1,3,4-octadecanetriol (S1140B-9)

Natural sponges sampled at 15 to 25 meters below the surface of the sea around the Kume Island, Okinawa, Japan were lyophilized to obtain powders. The obtained powders (447.1 g) were extracted with a mixed solution of chloroform and methanol, and the extract was concentrated under reduced pressure to obtain a 51.28 g of extract. This extract was fractionated with the aid of ethyl acetate and water. The upper layer and the intermediate layer were dried over anhydrous sodium sulfate, and they were concentrated under reduced pressure to obtain fractions of 18.37 g and 9.44 g, respectively. The fraction obtained from the upper layer was combined with an alcohol layer fractionated with the aid of aqueous 10% methanol and n-hexane and with the fraction obtained from the intermediate layer, and the resultant was concentrated. Thereafter, silica gel chromatography and normal-phase TLC were repeated, and thus, a single active component was obtained (169.9 mg). The active component was further purified by reversed-phase HPLC using an ODS-AM column (YMC, 250 mm x 20 mm (i.d.), methanol: 9.0 ml/min) (retention time: 30.3 min) to obtain a pure title compound (S1140B-9) (yield: 10.2 mg). The isolation and purification of the title compound can be conducted with reference to F. Cafieri et al., Liebigs Ann. Chem. 1995, pp. 1477-1481.
Negative FABMS: m/z 1007 [(M-H)⁻]
IR
¹H NMR (500 MHz, C₅D₅N, 24°C) δ (ppm) 8.55 (1H, d, J = 9.2 Hz, NH), 5.60 (1H, d, J = 3.7 Hz, H1"), 5.57 (1H, d, J = 3.7 Hz, H1'''), 5.13 (1H, m, H2), 4.75 (1H, dd, J = 3.7, 10.4 Hz, H2"), 4.62 (2H, m), 4.54 (4H, m), 4.25-4.47 (10H, m), 2.17 (2H, m), 1.99 (1H, m), 1.87 (2H, m), 1.75 (1H, m), 1.65 (2H, m), 1.12-1.49 (60H, m), 0.85 (6H, m, terminal methyl)
¹³C NMR (125 MHz, C₅D₅N, 45°C) δ (ppm) 175.5 (s, C1'), 99.5 (d, C1'''), 98.6 (d, C1"), 76.7 (d, C2"), 76.0 (d, C3), 72.8 (d, C4), 72.6 (d, C5"), 72.6 (d, C4"), 72.5 (d, C2), 71.3 (d, C3'''), 71.0 (d), 70.8 (d), 70.5 (d, C2'''), 69.7 (d, C3"), 68.6 (t, C1), 62.7 (t), 62.5 (t), 51.2 (t, C2), 39.4 (t), 35.6 (t), 33.7 (t), 32.2 (t), 30.5 (t), 30.3 (t), 30.1 (t), 30.0 (t), 29.7 (t), 29.6 (t), 26.7 (t), 26.0 (t), 23.0 (t), 22.9 (t), 14.3 (q, terminal methyl)

### [Example 3]

The following compounds were synthesized according to the processes described in literature as cited for each of the compounds:
(2S,3R)-1-(α-D-galactopyranosyloxy)-2-tetradecanoylamino-3-octadecanol (AGL-517) (WO 93/5055);
(2S,3R)-1-(α-D-glucopyranosyloxy)-2-tetradecanoylamino-3-octadecanol (AGL-563) (WO 94/9020);
(2S,3R)-1-(6'-deoxy-α-D-galactopyranosyloxy)-2-tetradecanoylamino-3-octadecanol (AGL-571) (WO 94/9020);
(2S,3R)-1-(β-L-arabinopyranosyloxy)-2-tetradecanoylamino-3-octadecanol (AGL-577) (WO 94/9020);
O-α-D-galactopyranosyl-(1→6)-O-α-D-galactopyranosyl-(1→1)-(2S,3S,4R)-2-amino-N-hexacosanoyl-1,3,4-octadecanetriol (AGL-586) (WO 94/24142);
O-α-D-galactopyranosyl-(1→6)-O-α-D-glucopyranosyl-(1→1)-(2S,3S,4R)-2-amino-N-hexacosanoyl-1,3,4-octadecanetriol (AGL-584) (WO 94/24142);
O-α-D-galactofuranosyl-(1→3)-O-α-D-galactopyranosyl-(1→1)-(2S,3S,4R)-2-amino-N-[(R)-2-hydroxytetracosanoyl]-1,3,4-octadecanetriol (719-7) (WO 94/24142); and
O-(N-acetyl-2-amino-2-deoxy-α-D-galactopyranosyl-(1→3)-O-[α-D-glucopyranosyl-(1→2)]-O-α-D-galactopyranosyl-(1→1)-(2S,3S,4R)-2-amino-N-[(R)-2-hydroxytetracosanoyl]-1,3,4-octadecanetriol (STL-8) (WO 94/24142).

Table 1 below shows the compounds represented by formula (I) which correspond to the compounds described in the Examples.

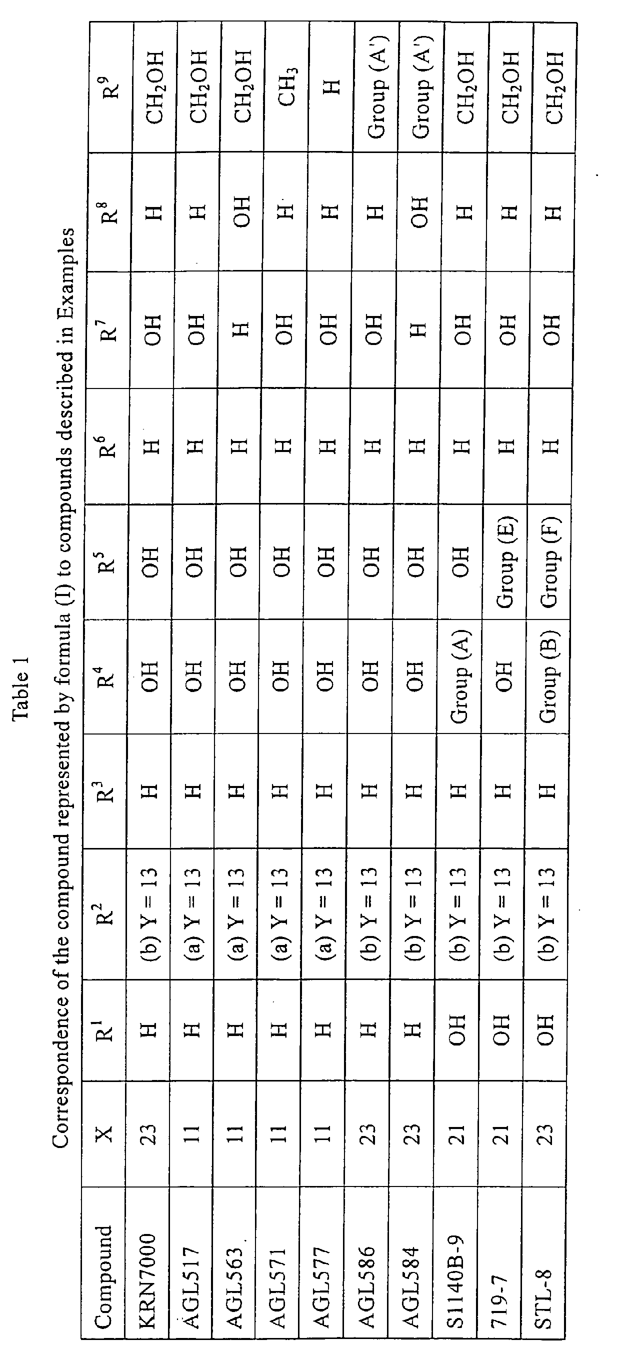

### [Example 4] Pharmacological test: Efficacy of KRN7000 on chimpanzees persistently infected with hepatitis C virus

The following experiment was carried out using the compound represented by formula (I) (KRN7000) as a representative example of the glycoside compound according to the present invention.

Chimpanzees are the only test animals that can be persistently infected with hepatitis C viruses. Thus, 2 chimpanzees were selected as test animals for this experiment from the chimpanzee-dedicated facility (Kumamoto Primates Park, Sanwa Kagaku Kenkyusho CO., Ltd.) where chimpanzees including hepatitis C virus carriers have been artificially bred and propagated over a long period of time. This experiment was examined and approved by the Experiment Review Board in advance in terms of ethics and test contents, and pain inflicted on chimpanzees was eliminated as much as possible during the experiment. Two chimpanzees employed in the experiment are hereafter referred to as Animal No. C33 (the duration of persistent infection with HCV (1a): 22 years) and Animal No. C54 (the duration of persistent infection with HCV (1b): 20 years), respectively.

After an acclimation period of approximately 1 month, KRN7000 was intravenously administered two times to animals at the rate of 10 µg/kg at the interval of 28 days (day 0 and day 28). In order to obtain samples for verifying the responses of chimpanzees to KRN7000 and efficacy of KRN7000 on chronic HCV infections, blood drawing was carried out before and after each administration. The total leukocyte count was measured using an automated blood cell counter Sysmex K4500 (Sysmex Corporation). The leukocyte percentage was determined by coating whole blood onto a fat-free slide glass, immobilizing it thereon, staining with Diff-Quick (International Reagents Corp.), and microscopically examining 200 leukocytes. Neutrophil and lymphocyte counts were calculated by multiplying the obtained values with the total leukocyte count. The NKT cell percentage in the T cells was determined by: staining the whole blood with an anti-human TCR Vα24 antibody (Beckman Coulter), KRN7000 (α-GalCer)-bound human CD1d tetramer, and an anti-human CD3 antibody (Becton Dickinson Japan) that are labeled with different fluorescent dyes; lysing the stained whole blood with the FACS Lysing Solution (Becton Dickinson Japan); immobilizing thereof; and then determining the NKT cell ratio using the FACSCalibur (Becton Dickinson Japan). Cells that had responded to all of the anti-human TCR Vα24 antibody, the tetramer, and the anti-human CD3 antibody were determined to be the NKT cells of the chimpanzees. The activities of alanine aminotransferase (ALT), aspartate aminotransferase (AST), and lactate dehydrogenase (LDH) in serums were assayed using an automatic analyzer. The serum HCV-RNA levels were assayed by RT-PCR using the AMPLICOR® GT HCV monitor (Roche Diagnostics) and branched DNA (bDNA) probe assay using the Quantiplex HCV-RNA 2 (Bayer Corporation). The serum KRN7000 levels were assayed by liquid chromatography and tandem mass spectrometry (LC-MS/MS).

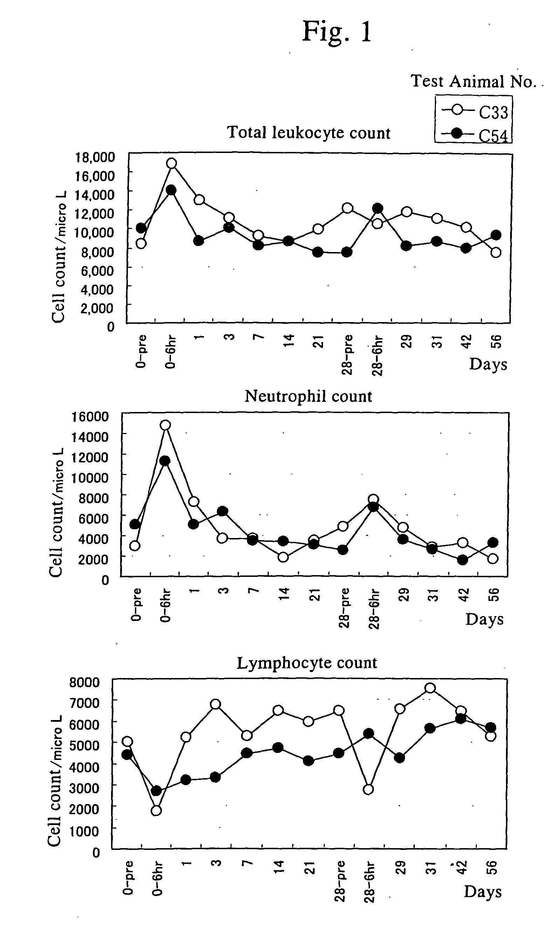

Fig. 1 shows changes in the total leukocyte count, the neutrophil count, and the lymphocyte count before and after the administration. In both stages of administration, the neutrophil counts of both chimpanzees increased 6 hours later. In many cases, the total leukocyte count transiently increased in such a manner that this increase reflected the changes in the neutrophil count. The lymphocyte count decreased 6 hours after the administration, although there were some exceptions.

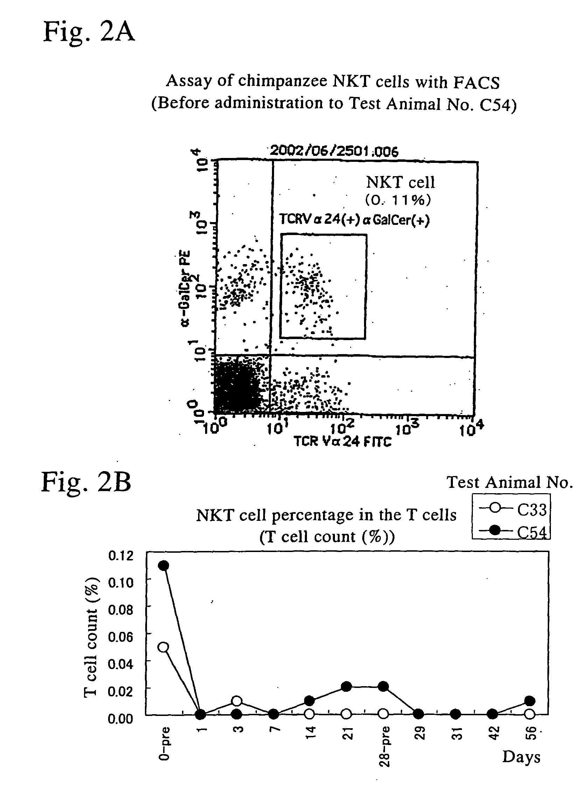

Fig. 2A is a typical diagram showing the results of analysis using the fluorescence activated cell sorter (FACS) on the tetramer-stained and TCR Vα24-positive NKT cells of chimpanzees that were present in the cell population gated with the CD3 positive cells. Fig. 2B shows changes in the NKT cell count before and after the administration of KRN7000. NKT cells were not detected on the day following both administrations in both chimpanzees. In the case of Test Animal No. C54, NKT cells gradually showed a tendency to recover after they had disappeared after the administration of KRN7000. Rapid disappearance of NKT cells after the administration of KRN7000 indicated that apoptosis occurred immediately after the activation of NKT cells via KRN7000 stimulation. Various types of cytokines are deduced to be released from the activated NKT cells.

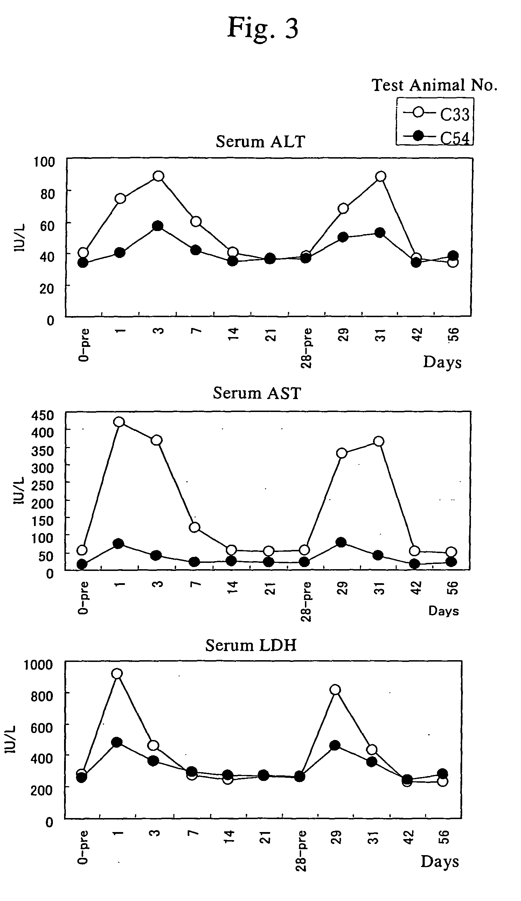

Fig. 3 shows the levels of serum ALT, AST, and LDH activities before and after the administration of KRN7000, which can be clinical indicators of liver dysfunction. These levels were transiently and mildly elevated, reaching peaks 1 day or 3 days after the administration in both chimpanzees in both stages of administrations. Thereafter, these values subsequently returned to levels before the administration.

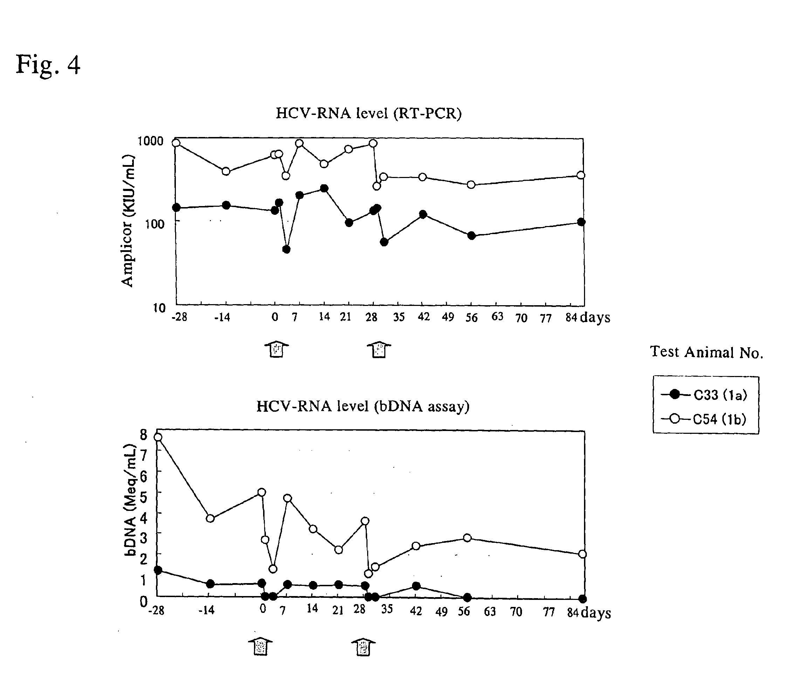

Fig. 4 shows changes in the serum HCV-RNA levels before and after the administration of KRN7000 as assayed by RT-PCR or bDNA technique. Both chimpanzees exhibited apparent decreases in the serum HCV-RNA levels 1 day or 3 days after the administration of KRN7000 in both stages. The HCV-RNA levels were continuously decreased by the second administration (day 28) than by the initial administration (day 0).

Table 2 shows the assay results of the serum KRN7000 levels before and after the administration of KRN7000. The maximum levels of KRN7000 were detected 15 minutes after the administration of KRN7000 in both chimpanzees in both stages. This indicates that the administered KRN7000 actually migrated in the bodies.

**Table 2**

| Assay of serum KRN7000 levels before and after the administration | | | |
|---|---|---|---|
| Test Animal No. | Stage of administration | Timing of blood sampling | Concentration in blood (ng/ml) |
| C33 | First | Before administration | < 10 |
| | | 15 minutes later | 89 |
| | | 6 hours later | 38 |
| | Second | Before administration | < 10 |
| | | 15 minutes later | 97 |
| | | 6 hours later | 58 |
| C54 | First | Before administration | < 10 |
| | | 15 minutes later | 129 |
| | | 6 hours later | 42 |
| | Second | Before administration | < 10 |
| | | 15 minutes later | 141 |
| | | 6 hours later | 77 |

Changes in the leukocyte count and mild and transient liver dysfunctions, that could result from released cytokines upon activation of NKT cells stimulated by the administration of KRN7000, were observed, and the serum HCV-RNA levels also decreased. This indicates that replication of HCV-RNA was locally inhibited in HCV-infected liver cells by the action of the KRN7000 administered. Accordingly, the present experiment revealed that KRN7000 had anti-HCV effects on chimpanzees infected with chronic HCV.

All publications, patents, and patent applications cited herein are incorporated herein by reference in their entirety.

### Industrial Applicability

As described in the Examples, administration of the agent according to the present invention to chimpanzees infected with hepatitis C viruses can decrease the levels of hepatitis C viruses and enhance the liver functions that have been adversely affected by the infections. Accordingly, the agent of the present invention can be utilized as an inhibitor of hepatitis C virus or therapeutic agent of hepatitis C for humans.

## Claims

1. An inhibitor of hepatitis C viruses comprising, as an active ingredient, a compound represented by formula (I) or a salt or solvate thereof: 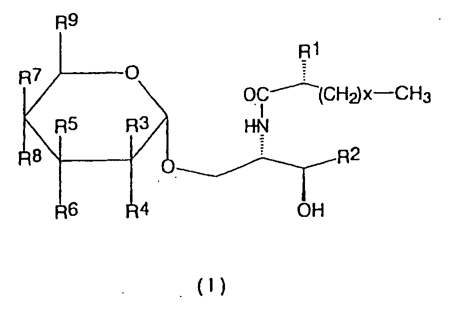 wherein
R¹ represents H or OH;
X is an integer between 7 and 27;
R² is any of substituents (a) to (e) below (wherein Y is an integer between 5 and 17):
(a) -CH₂(CH₂)_{Y}CH₃;
(b) -CH(OH)(CH₂)_{Y}CH₃;
(c) -CH(OH)(CH₂)_{Y}CH(CH₃)₂;
(d) -CH=CH(CH₂)_{Y}CH₃; or
(e) -CH(OH)(CH₂)_{Y}CH(CH₃)CH₂CH₃; and
R³ to R⁹ are independently a substituent defined by any of i) to v) below:
i) when R³, R⁶, and R⁸ independently represent H, R⁴ is H, OH, NH₂, NHCOCH₃, or a substituent defined by any of (A) to (D) below: 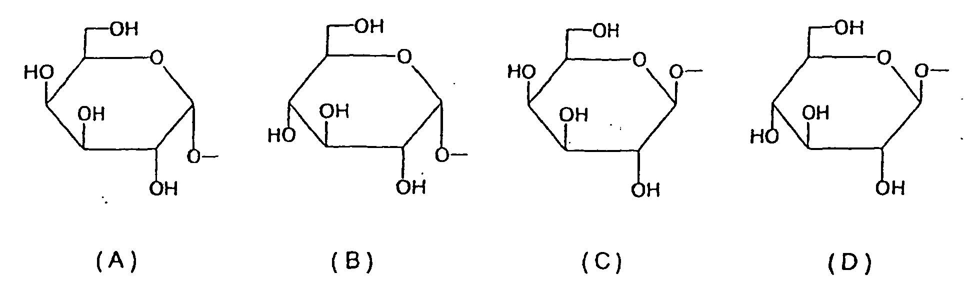 R⁵ is OH or a substituent defined by (E) or (F) below: 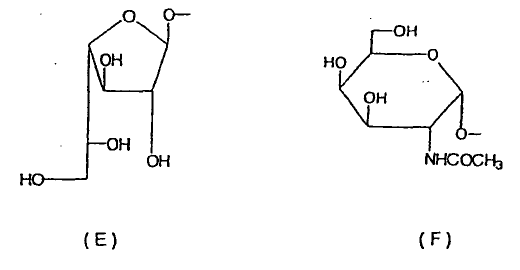 R⁷ is OH or a substituent defined by any of (A) to (D) below: 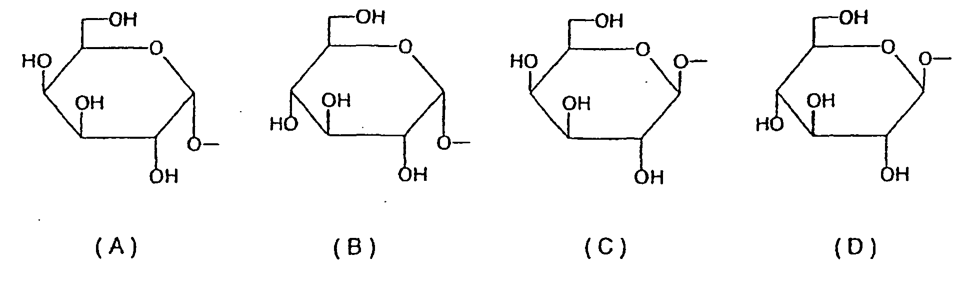 R⁹ is H, CH₃, CH₂OH, or a substituent defined by any of (A') to (D') below: 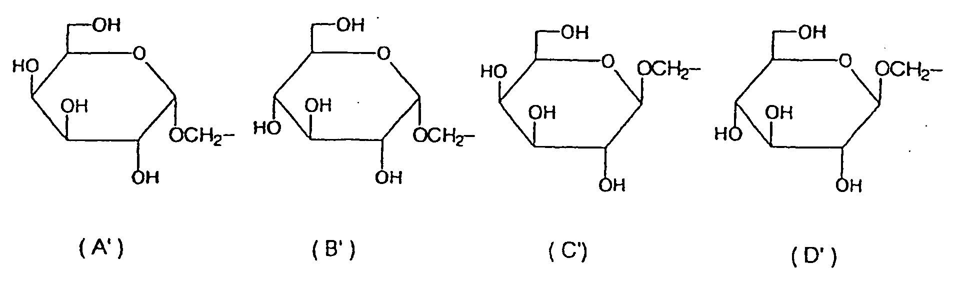 or
ii) when R³, R⁶, and R⁷ are independently H, R⁴ is H, OH, NH₂, NHCOCH₃ or a substituent defined by any of (A) to (D) below: 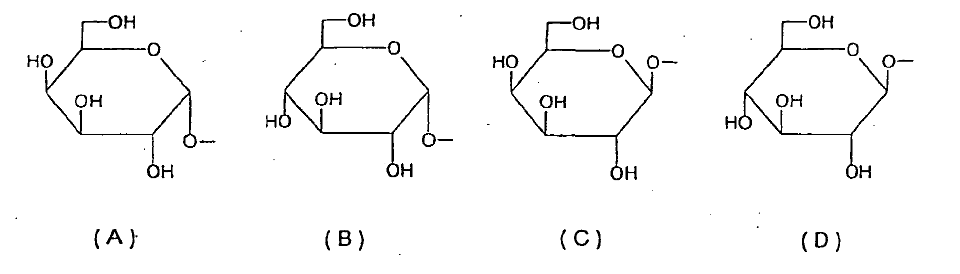 R⁵ is OH or a substituent defined by (E) or (F) below: 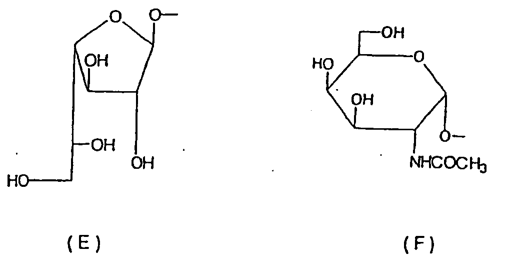 R⁸ is OH or a substituent defined by any of (A) to (D) below: 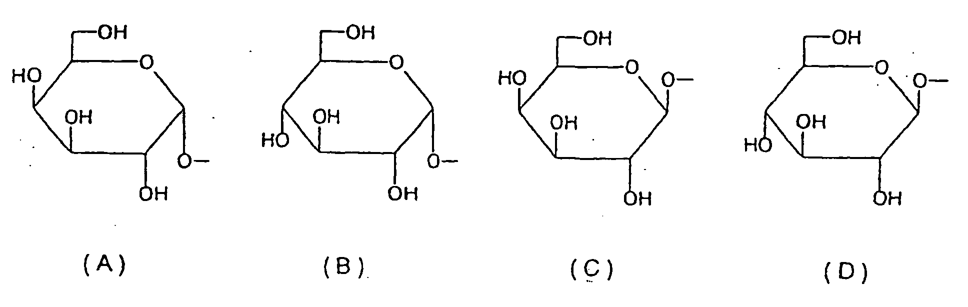 and
R⁹ is H, CH₃, CH₂OH or a substituent defined by any of (A') to (D') below:

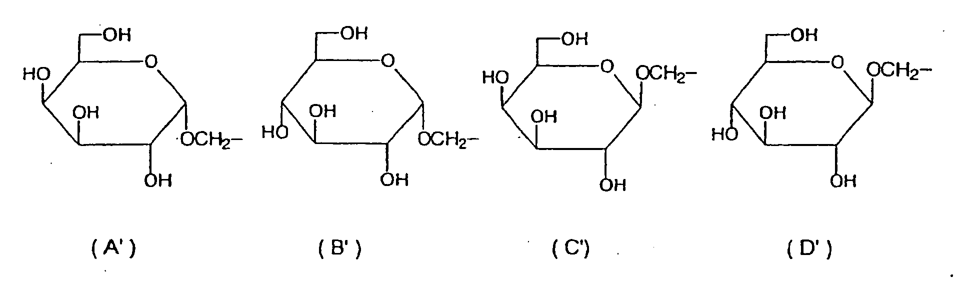

2. The inhibitor of hepatitis C viruses according to claim 1, wherein the hepatitis C virus is genotype 1.

3. A therapeutic agent for hepatitis C comprising, as an active ingredient, the compound represented by formula (I) according to claim 1 or a salt or solvate thereof.

4. The therapeutic agent for hepatitis C according to claim 3, wherein hepatitis C is chronic hepatitis C.

5. The therapeutic agent for hepatitis C according to claim 3, wherein hepatitis C is acute hepatitis C.

6. An agent for enhancing liver functions adversely affected due to hepatitis C comprising, as an active ingredient, the compound represented by formula (I) according to claim 1 or a salt or solvate thereof.

7. The agent according to any one of claims 1 to 6, wherein, in a compound represented by formula (I), R³ and R⁶ are independently H, R⁴ is OH or a substituent defined by any of (A) to (D), R⁵ is OH or a substituent defined by (E) or (F), R⁷ and R⁸ are independently H or OH, provided that R⁷ and R⁸ do not simultaneously represent the same group, and R⁹ represents CH₂OH, CH₃, H, or a substituent defined by any of (A') to (D').

8. The agent according to any one of claims 1 to 6, wherein, in a compound represented by formula (I), X is an integer between 21 and 25 and R² is a substituent (b) (wherein Y is an integer between 11 and 15).

9. The agent according to any one of claims 1 to 6, wherein, in a compound represented by formula (I), X is an integer between 9 and 13 and R² is a substituent (a) (wherein Y is an integer between 11 and 15).

10. The agent according to any one of claims 1 to 6, wherein a compound represented by formula (I) is selected from the group consisting of:
(2S,3S,4R)-1-(α-D-galactopyranosyloxy)-2-hexacosanoylamino-3,4-octadecanediol;
(2S,3R)-1-(α-D-galactopyranosyloxy)-2-tetradecanoylamino-3-octadecanol;
(2S,3R)-1-(α-D-glucopyranosyloxy)-2-tetradecanoylamino-3-octadecanol;
(2S,3R)-1-(6'-deoxy-α-D-galactopyranosyloxy)-2-tetradecanoylamino-3-octadecanol;
(2S,3R)-1-(β-L-arabinopyranosyloxy)-2-tetradecanoylamino-3-octadecanol;
O-α-D-galactopyranosyl-(1→6)-O-α-D-galactopyranosyl-(1→1)-(2S,3S,4R)-2-amino-N-hexacosanoyl-1,3,4-octadecanetriol;
O-α-D-galactopyranosyl-(1→6)-O-α-D-glucopyranosyl-(1→1)-(2S,3S,4R)-2-amino-N-hexacosanoyl-1,3,4-octadecanetriol;
O-α-D-galactopyranosyl-(1→2)-O-α-D-galactopyranosyl-(1→1)-(2S,3S,4R)-2-amino-N-[(R)-2-hydroxytetracosanoyl]-1,3,4-octadecanetriol;
O-β-D-galactofuranosyl-(1→3)-O-α-D-galactopyranosyl-(1→1)-(2S,3S,4R)-2-amino-N-[(R)-2-hydroxytetracosanoyl]-1,3,4-octadecanetriol; and
O-(N-acetyl-2-amino-2-deoxy-α-D-galactopyranosyl-(1→3)-O-[α-D-glucopyranosyl-(1→2)]-O-α-D-galactopyranosyl-(1→1)-(2S,3S,4R)-2-amino-N-[(R)-2-hydroxytetracosanoyl]-1,3,4-octadecanetriol.

11. The agent according to any one of claims 1 to 6, wherein a compound represented by formula (I) is selected from the group consisting of:
(2S,3S,4R)-1-(α-D-galactopyranosyloxy)-2-hexacosanoylamino-3,4-octadecanediol;
O-α-D-galactopyranosyl-(1→6)-O-α-D-galactopyranosyl-(1→1)-(2S,3S,4R)-2-amino-N-hexacosanoyl-1,3,4-octadecanetriol;
O-α-D-galactopyranosyl-(1→6)-O-α-D-glucopyranosyl-(1→1)-(2S,3S,4R)-2-amino-N-hexacosanoyl-1,3,4-octadecanetriol;
O-α-D-galactopyranosyl-(1→2)-O-α-D-galactopyranosyl-(1→1)-(2S,3S,4R)-2-amino-N-[(R)-2-hydroxytetracosanoyl]-1,3,4-octadecanetriol;
O-β-D-galactofuranosyl-(1→3)-O-α-D-galactopyranosyl-(1→1)-(2S,3S,4R)-2-amino-N-[(R)-2-hydroxytetracosanoyl]-1,3,4-octadecanetriol; and
O-(N-acetyl-2-amino-2-deoxy-α-D-galactopyranosyl-(1→3)-O-[α-D-glucopyranosyl-(1→2)]-O-α-D-galactopyranosyl-(1→1)-(2S,3S,4R)-2-amino-N-[(R)-2-hydroxytetracosanoyl]-1,3,4-octadecanetriol.

12. The agent according to any one of claims 1 to 6, wherein a compound represented by formula (I) is (2S,3S,4R)-1-(α-D-galactopyranosyloxy)-2-hexacosanoylamino-3,4-octadecanediol.
